(19) Europäisches Patentamt

European Patent Office

Office européen des brevets

(11) Veröffentlichungsnummer: **0 280 762 B1**

# (12) EUROPÄISCHE PATENTSCHRIFT

(45) Veröffentlichungstag der Patentschrift: **21.10.92**

(21) Anmeldenummer: **87112084.6**

(22) Anmeldetag: **20.08.87**

(51) Int. Cl.⁵: **C12M 1/00**

(54) **Verfahren und Anlage zur Herstellung von Biogas aus dickflüssigen vergärbaren Medien.**

(30) Priorität: **02.03.87 DE 3706699**

(43) Veröffentlichungstag der Anmeldung:
**07.09.88 Patentblatt 88/36**

(45) Bekanntmachung des Hinweises auf die
Patenterteilung:
**21.10.92 Patentblatt 92/43**

(84) Benannte Vertragsstaaten:
**AT BE CH DE FR GB LI LU NL SE**

(56) Entgegenhaltungen:
**CH-A- 657 150**
**DE-A- 3 102 739**
**FR-A- 994 032**

**CHEMIKER ZEITUNG, Band 108, Nr. 5, 1984, Seiten 165-172, Heidelberg, DE; E.A. STAD-BAUER et al.: "Biogasanlagen: Nutzung des mikrobiellen Lebensraums bei der Entsorgung"**

**PROGRESS IN ENERGY AND COMBUSTION SCIENCE, Band 8, Nr. 2, 1982, Seiten 135-157, Pergamon Press Ltd, Oxford, GB; P.N. HOBSON et al.: "Production and use of biogas in agriculture"**

(73) Patentinhaber: **Frese, Christoph**
**Twengweg 13**
**W-5789 Medebach 8(DE)**

(72) Erfinder: **Frese, Christoph**
**Twengweg 13**
**W-5789 Medebach 8(DE)**

(74) Vertreter: **Fritz, Herbert, Dipl.-Ing.**
**Mühlenberg 74**
**W-5760 Arnsberg 1(DE)**

EP 0 280 762 B1

**Beschreibung**

Die vorliegende Erfindung betrifft ein Verfahren zur Herstellung von Biogas durch Vergärung dickflüssiger Medien, bei dem die Vergärung in einem Gärbehälter in zwei Stufen erfolgt, nämlich einer ersten Säuregärungsstufe, bei der mit Hilfe von Säurebakterien Sauerstoff verbraucht wird und einer anschließenden zweiten Hauptgärungsstufe, bei der das Biogas zum Beispiel mit Methanbakterien erzeugt wird. Die Erfindung betrifft weiterhin eine Anlage zur Herstellung von Biogas durch Vergärung dickflüssiger Medien, bei der in einem vorzugsweise zylindrischen Behälter vergoren wird.

Es ist aus dem Stand der Technik, z.B. aus der DE-C 31 02 739, bekannt, die Vergärung in zwei aufeinanderfolgenden Stufen in zwei voneinander getrennten Gärkammern durchzuführen. Das Gärmedium gelangt zunächst in eine erste Gärkammer, in der die Vorgärung mit Hilfe von Säurebakterien durchgeführt wird, die sogenannte Säuregärung, bei der unter anderem der im Gärmedium vorhandene Sauerstoff verbraucht wird, da die bei der Hauptgärung eingesetzten Methanbakterien bei Ausschluß von Sauerstoff bessere Lebensbedingungen vorfinden. In der Säuregärungsstufe werden außerdem die im Gärmedium vorhandenen hochmolekularen Eiweißstoffe, Kohlenhydrate und Fette in niedermolekulare Stoffe, insbesondere Fettsäuren abgebaut. Als Stoffwechselprodukt der Säurebakterien entstehen bei der Säuregärung im wesentlichen $CO_2$, $H_2$ und $H_2S$. Anschließend gelangt das Gärmedium aus dem Säuregärraum in den eigentlichen Hauptgärraum, in dem die Methanbakterien aus den Abbauprodukten der ersten Stufe das Biogas erzeugen. Sofern eine Vorrichtung für die Durchmischung des Gärmediums vorgesehen ist, wird diese in beiden Gärräumen durch herkömmliche Rührer vorgenommen, zum Beispiel bei Verwendung zylindrischer Gärbehälter durch Rührer, deren Drehachse die vertikale Behälterachse ist, wobei Teile des Rührers von dieser Achse in radialer Richtung nach außen ragen. Bei Drehung der Rührer wird demnach das Gärmedium in eine Rotationsbewegung um die Behältermittelachse versetzt. Da in dem Gärmedium auch Feststoffanteile enthalten sind, nimmt die Wirkung des Rührers und der Grad der Durchmischung von der Behälterachse nach außen hin ab. Die bei der Gärung eingesetzten Bakterien sind gegen starke Strömungen im Gärmedium empfindlich. Es ist deshalb nicht angebracht, eine bessere Durchmischung bei einem solchen Rührer durch Erhöhung der Rührgeschwindigkeit zu erzielen. Findet die Gärung in zwei getrennten Gärkammern statt, ist für jede Gärkammer ein Rührwerk erforderlich. Außerdem besteht die Gefahr, daß das Gärmedium, das in der Regel durch Öffnungen in der Behälterwand oder im Behälterboden in diesen gelangt, den Behälter nach zu kurzer Verweilzeit vorzeitig durch den Auslauf verläßt. Um eine vollständige Durchgärung zu erhalten, ist es jedoch erstrebenswert, eine möglichst gleichmäßige Vermischung des frischen zugeführten mit dem angegorenen Gärmedium zu erhalten.

Die Aufgabe der vorliegenden Erfindung besteht demnach darin, ein Verfahren der eingangs genannten Art dahingehend zu verbessern, daß eine möglichst gleichmäßige langsame kontinuierliche Vermischung des Gärmediums in sämtlichen Bereichen des Behälters stattfindet, ohne daß dabei die Bakterien irritierende starke Strömungen oder Turbulenzen im Gärmedium auftreten, so daß die Biogas-Ausbeute maximiert und die Verweildauer im Behälter minimiert wird. Die Lösung dieser Aufgabe liefert ein Verfahren, bei dem in beiden Gärungsstufen die Durchmischung durch langsame kontinuierliche Bewegung von etwa plattenförmigen Mischkörpern im Behälter in axialer Richtung erfolgt. Sofern es sich um einen stehenden Behälter handelt, werden die Mischkörper, die sich nahezu über den gesamten Querschnitt des Behälters erstrecken, im Behälter auf- und abbewegt. Ebensogut ist auch denkbar, den Behälter liegend auszuführen, so daß in diesem Fall die plattenförmigen Mischkörper im Behälter stehend, den Behälterquerschnitt im wesentlichen abdeckend, entlang der Behälterachse bewegt werden.

In der Beschreibung der DE-C 31 02 739 wird davon ausgegangen, daß die erste Stufe der Säuregärung ausschließlich anaerob zu erfolgen hat. Es hat sich jedoch erfindungsgemäß gezeigt, daß dies keineswegs erforderlich ist. Vielmehr kann die erste Säuregärungsstufe sowohl anaerob als auch aerob erfolgen, wobei in letzterem Fall Sauerstoff in den Behälterabschnitt, in dem die Säuregärung stattfindet, eingetragen werden muß. Es hat sich nun gezeigt, daß bei somit aerober Durchführung der Säuergärungsstufe die Ausbeute an Biogas sehr gut ist, wenn bestimmte Verfahrensbedingungen eingehalten werden. Wichtig ist dabei insbesondere, daß der Sauerstoff, der in der Regel in Form von Luft eingebracht wird, die Säuregärungsbakterien möglichst an allen Stellen innerhalb der Gärkammer erreicht, das heißt, daß das Gärmedium wirklich gleichmäßig belüftet wird. Es ist deshalb erfindungsgemäß vorgesehen, den Eintrag des Sauerstoffs (der Luft) über die Mischkörper vorzunehmen, die eine große Oberfläche aufweisen und durch die Bewegung das Gärmedium überall im Behälter erreichen.

Für die Durchführung des zweistufigen Gärprozesses ist die Wahl der optimalen Betriebstemperatur wichtig, die innerhalb der Gärkammern weitgehend gleichbleibend sein sollte. Die Bakterien sind gegen Temperaturschwankungen sehr empfindlich und tolerieren meist nur geringe Schwankungen. Nach dem Stand der Technik wird das Gärmedium entweder warm in den Behälter eingeführt oder im Behälter durch Fußboden- oder Wandheizung oder auch durch Wärmetauscher angewärmt. Bei Heizung des Bodens oder der Wand des Behälters geht ein Teil der Wärme durch die Behälterwandung verloren. Darüberhinaus entweicht auch Wärme mit dem ausströmenden Biogas. Es ist daher erfindungsgemäß vorgesehen, die ausschließliche oder zumindest eine zusätzliche Aufwärmung des Gärmediums durch Erwärmen der Mischkörper vorzunehmen. Da sich die Mischkörper im Gärmedium kontinuierlich auf- und abbewegen, wird dieses auch überall gleichmäßig erwärmt. Da die Mischkörper in das Gärmedium eintauchen, sind die Wärmeverluste über Behälterboden und Wandung gering. Dadurch, daß über die Mischkörper selbst erwärmt wird, indem man z.B. Warmwasser durch diese leitet, genügt es, wenn das Wasser gegenüber dem Gärmedium eine jeweils nur geringfügig höhere Vorlauftemperatur aufweist. Dagegen war bei Heizung der Behälterwandung nach dem Stand der Technik eine wesentlich höhere Vorlauftemperatur erforderlich, was wärmetechnisch ungünstig ist. Gemäß einer bevorzugten Ausführungsform der Erfindung kann vorgesehen sein, das Gärmedium vor der Zufuhr in den Säuregärraum mittels eines Wärmetauschers vorzuwärmen, wobei eine Vorwärmung auf etwa 20° - 25°C angebracht ist.

Für das erfindungsgemäße Verfahren ist es also ohne Belang, ob die erste Gärungsstufe mit Hilfe der Säurebakterien aerob oder anaerob verläuft. Es versteht sich jedoch, daß die anschließende Methangärung unter Ausschluß von Sauerstoff erfolgen muß. Bei aerober Säuregärung muß daher die Säuregärkammer von der Methangärkammer abgetrennt werden. Bei der aeroben Säuregärung kann es vorkommen, daß aufgrund der bakteriellen Abbauprozesse sich das Gärmedium über die gewünschte Temperatur hinaus erwärmt. In diesem Fall ist erfindungsgemäß vorgesehen, die überschüssige Wärme über die Mischkörper abzuführen, z.B. indem man durch die Mischkörper Kaltwasser leitet, und somit das Gärmedium zu kühlen. Auch hier ist es wieder von Vorteil, daß die Mischkörper das Gärmedium überall im Behälter erreichen, da die Bakterien gegen Überhitzung empfindlich sind.

Nach dem erfindungsgemäßen Verfahren können alle dickflüssigen Medien vergoren werden, insbesondere ist das Verfahren für die Vergärung von Gülle oder Klärschlamm gut geeignet.

Da beispeilsweise in einem landwirtschaftlichen Betrieb ständig Gülle anfällt und die Verweildauer der Gülle im Gärbehälter weitgehend konstant sein sollte, ist eine kontinuierliche Zufuhr der Gülle, z.B. über einen Zeitraum von 24 Stunden, sinnvoll. Hierfür hat sich erfindungsgemäß eine pneumatische Fördereinrichtung z.B. eine mit Druckluft betätigte spezielle Pumpe als gut geeignet erwiesen, mit deren Hilfe die Gülle aus einem Vorratsbehälter schubweise in den Gärbehälter gepumpt wird. Die von einem Kompressor erzeugte Druckluft hat als Pumpmedium den Vorteil, daß sie sehr genau dosiert werden kann. Des weiteren enthält diese Pumpe keine beweglichen Teile, durch die die Gülle aufgewühlt wird, so daß für die Bakterien schädliche Turbulenzen vermieden werden. Die Pumpe ist Gegenstand einer gesonderten Anmeldung des Anmelders. Damit gegebenenfalls eine Übersäuerung des Gärmediums verhindert werden kann, wird der pH-Wert des Gärmediums beim Verlassen der Säuregärungsstufe gemessen.

Weiter ist Gegenstand der Erfindung eine Anlage zur Herstellung von Biogas durch Vergärung dickflüssiger Medien mit einem vorzugsweise zylindrischen Behälter, in dem das Gärmedium vergoren wird. Die Durchmischung des Gärmediums erfolgt durch plattenförmige Mischkörper, die zum Beispiel Betonplatten sein können, und die im Behälter in axialer Richtung bewegt werden. Bei einem stehenden Gärbehälter können die Mischkörper im Behälter aufgehängt werden und in vertikaler Richtung angehoben bzw. abgesenkt werden. Das Anheben der Mischkörper kann zum Beispiel durch Aufwickeln der Aufhängungsseile auf eine oben im Behälter angebrachte um ihre horizontale Achse drehbare Walze erfolgen. Wenn, wie vorzugsweise vorgesehen, die Vergärung der Gülle in zwei Stufen erfolgt, einer ersten Säuregärungsstufe und einer sich daran anschließenden Hauptgärungsstufe, kann der Gärbehälter zum Beispiel aus einer inneren ringförmigen Säuregärungskammer bestehen, die ein Gasentlüftungsrohr aufweist, durch das die entstehenden nicht brauchbaren Gase, hauptsächlich $CO_2$, $H_2$ und $H_2S$ entweichen können. Die Vorschaltung der Säuregärung ist insbesondere bei der Verarbeitung von Rindergülle vorteilhaft, da deren pH-Wert recht hoch liegt. Da die Säurebakterien Säure entwickeln, wird somit der pH-Wert gesenkt. Um für die Methanbakterien der Hauptgärung gute Lebensbedingungen zu erhalten, wird der pH-Wert beim Verlassen der Säuregärung kontrolliert. Durch eine Öffnung in Nähe des Behälterbodens ist die Säuregärungskammer mit dem Hauptgärraum verbunden, in dem die Methanbakterien das Biogas entwickeln. Im oberen Bereich des Hauptgärraums befindet sich die Abführleitung für das Biogas. Dabei ist es vorteilhaft, wenn man die Biogasleitung durch den Behälter hindurchführt, da so vermieden wird, daß die Biogasleitung durch die Gase mit einem relativ hohen Feuchtigkeitsgehalt geleitet werden, im Winter einfriert.

Gemäß einer Variante der Erfindung kann der Behälter für die erfindungsgemäße Biogasanlage auch so ausgebildet sein, daß die eine Behälterhälfte als Säuregärungskammer und die andere Behälterhälfte als Hauptgärkammer dient, wobei dann die Säuregärkammer von der Hauptgärkammer durch eine vertikale Trennwand abgeschlossen ist. Außerdem ist die Säuregärkammer nach oben hin durch eine Verschlußdecke abgeschlossen. Zwischen den beiden Gärkammern besteht vorzugsweise in Bodennähe des Behälters eine Verbindungsöffnung in der Trennwand, durch die die Gülle beim Verlassen der Säuregärungskammer in die andere Gärkammer gelangen kann. Dabei hat es sich im Gegensatz zu der aus der DE-C 31 02 739 bekannten Biogasanlage, erfindungsgemäß als vorteilhaft erwiesen, die beiden Gärkammern etwa gleich groß auszubilden. Der Ablauf der Säuregärung kann bei der erfindungsgemäßen Anlage zum einen über die Kontrolle der Temperatur, das heißt je nach Bedarf Erwärmung oder Kühlung des Gärmediums, und zum anderen bei der aeroben Säuregärung über den Lufteintrag in das Gärmedium kontrolliert werden. Dabei hat sich gezeigt, daß durch einen besseren Abbau der hochmolekularen Stoffe in der Säuregärungsstufe die Biogasausbeute in der anschließenden Methangärung erhöht wird. Somit erscheint es nicht zweckmäßig, im Reaktorraum eine volumenmäßige Aufteilung von Säurephase zu Methanphase im Verhältnis 1:10 vorzunehmen. Es sei an dieser Stelle jedoch betont, daß die erfindungsgemäße Anlage sowohl für ein Verfahren zur Erzeugung von Biogas mit als auch ohne vorgeschaltete Säuregärung eingesetzt werden kann, und daß, wenn eine Säuregärung vorgesehen ist, diese anaerob oder aerob erfolgen kann. Die erfindungsgemäße Anlage, bei der es auf die Art der Durchmischung des Gärmediums ankommt, ist also in der Anwendung nicht auf ein Verfahren gemäß Anspruch 1 dieser Anmeldung beschränkt.

In bevorzugter Weise ist vorgesehen, daß sowohl die Mischkörper für die innere Säuregärungskammer als auch die Mischkörper für den Hauptgärraum mit Seilen verbunden sind, die sich auf ein und dieselbe Walze aufwickeln, so daß die Mischkörper, die für die Durchmischung in beiden Kammern sorgen, durch Antrieb der Walze gleichzeitig bewegt werden können. Die Mischkörper für den Hauptgärraum, in der Regel zwei gleichgroße Betonplatten, sind dabei zum Beispiel mit Drahtseilen an der Walze so aufgehängt, daß sich bei deren Drehung die Mischkörper jeweils gegenläufig bewegen, so daß die Antriebskraft für die Walze gering ist. Während sich der eine Mischkörper durch Abwicklung des Seils von der Walze langsam nach unten bewegt, bewegt sich der andere Mischkörper in der anderen Behälterhälfte nach oben, wobei sich dessen Befestigungsseile auf die Walze aufwickeln. Die Verwendung von Mischkörpern aus Beton hat den Vorteil, daß diese korrosionsfest sind. Man kann auch ohne weiteres bereits im Betrieb befindliche herkömmliche Behälter zur Herstellung von Biogas mit den erfindungsgemäßen Mischkörpern nachrüsten. Die Mischkörper können dabei wegen des hohen Gewichts im Behälter gegossen werden, so daß der Transport entfällt. Es hat sich als vorteilhaft erwiesen, die Mischkörper mit Drahtseilen an jeweils drei Punkten mit jeweils drei Seilen aufzuhängen, so daß sich beim Drehen der Walze die Mischkörper reibungslos ohne die Gefahr der Verkantung im Behälter auf- und abbewegen können. Für eine bessere Durchmischung des Gärmediums ist es vorteilhaft, die Mischkörper so aufzuhängen, daß diese um einige Grad zur Behältermittelachse hin geneigt sind. Gemäß einer bevorzugten Ausführungsform der erfindungsgemäßen Anlage erfolgt die Aufheizung des Gärmediums über die Mischkörper, in denen z. B. nach Art einer Fußbodenheizung Warmwasserrohre verlegt sind. Auf diese Weise wird eine sehr gleichmäßige Erwärmung erreicht, bei der keine Temperaturgradienten im Behälter, z.B. von innen nach außen, auftreten. Außerdem werden so die Wärmeverluste über Behälterwandung und -boden minimiert. Die erfindungsgemäß verwendeten plattenförmigen Mischkörper haben außerdem den weiteren Vorteil, daß diese bei ihrer axialen Bewegung durch den Behälter das Gärmedium so durchmischen, daß die einzelnen Schichten in ihren Ausfaulphasen erhalten bleiben und nicht Gärmedium verschiedener Gärphasen durchmischt wird. Das ausgegorene Gärgut wird außerdem schichtweise quantitativ aus dem Behälter verdrängt. Es wird somit verhindert, daß im Bereich der Säuregärung in Folge der auch dort vorhandenen Methanbakterien bei dem säurevergorenen Gärgut bereits eine Methangärung stattfindet (sogenannte Mischgärung). Diese Gefahr bestünde sonst besonders bei Gärgut mit hohem Feststoffanteil. Die weiteren in den Unteransprüchen genannten Merkmale der erfindungsgemäßen Anlage haben vorteilhafte Weiterbildungen der Erfindung zum Inhalt und werden im einzelnen anhand der Figurenbeschreibung erläutert.

Im folgenden wird die vorliegende Erfindung anhand eines Ausführungsbeispiels unter Bezugnahme auf die Zeichnungen näher erläutert. Dabei zeigen

Fig. 1 einen Längsschnitt durch eine erfindungsgemäße Anlage zur Herstellung von Biogas aus Gülle;

Fig. 2 einen horizontalen Schnitt durch die Anlage gemäß Fig. 1;

Fig. 3 einen Schnitt durch einen Mischkör-

Fig. 4 eine vergrößerte Ansicht der Walze für die Aufhängung der Mischkörper;

Fig. 5 eine Seitenansicht in Richtung des Pfeiles IV von Fig. 4;

Fig. 6 einen vereinfachten Längsschnitt durch einen erfindungsgemäßen Behälter mit Darstellung der Strömungsverhältnisse;

Fig. 7 einen Längsschnitt durch eine Anlage gemäß einer Variante der Erfindung mit aerober Säuregärung;

Fig. 8 einen horizontalen Schnitt durch die Anlage gemäß Fig. 7;

Fig. 9 eine vergrößerte Schnittdarstellung eines Mischkörpers.

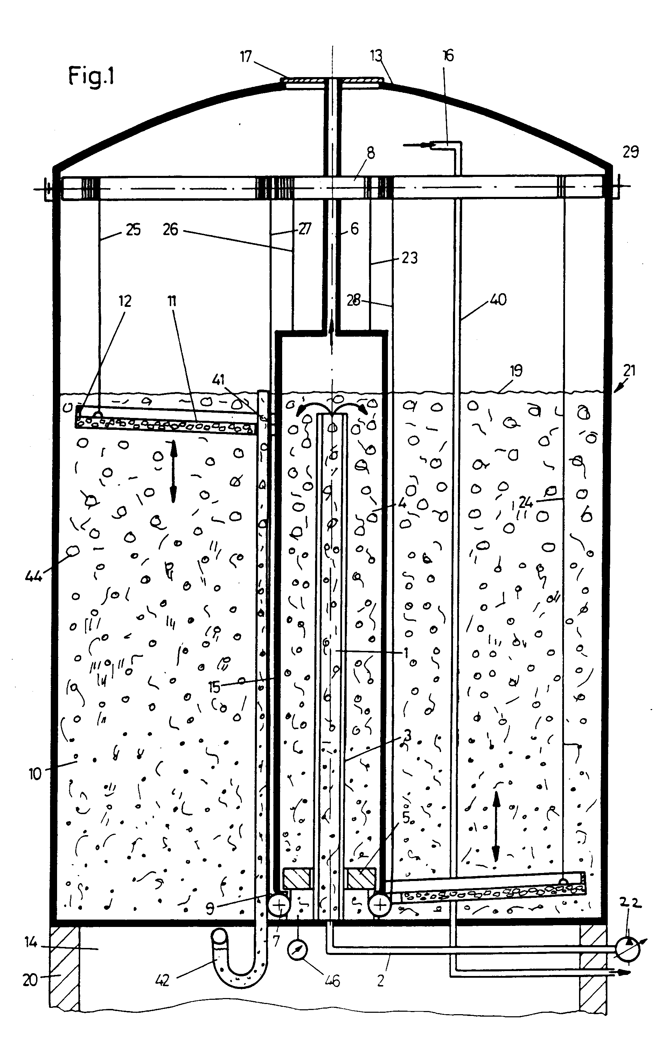

Zunächst wird auf Fig. 1 Bezug genommen. Bei der dargestellten Anlage zur Herstellung von Biogas aus Gülle ist ein kreiszylindrischer Behälter 21 vorgesehen, der auf einem Fundament 20 steht. Die Gülle wird mittels einer Pumpe 22, vorzugsweise mit Hilfe von mittels eines Kompressors erzeugter Druckluft über die Zuleitung 2 von unten in den Behälter gepumpt. Dabei gelangt die Gülle zunächst in eine innere zylindrische Aufwärmzone 1, deren Wandung von einem Wärmetauscher 3 gebildet wird.

Der Wärmetauscher 3 ist ein doppelwandiger Raum, durch den zum Beispiel Warmwasser geleitet wird. Das Einpumpen der Gülle erfolgt relativ langsam, und zwar schubweise, jedoch wird ständig Frischgülle in den Behälter gepumpt, während verbrauchte Gülle ständig den Behälter verläßt, so daß die Anlage insgesamt kontinuierlich betrieben wird. Wenn zum Beispiel in einem landwirtschaftlichen Betrieb pro Tag 10 m³ Gülle anfallen, werden stündlich gut 0,4 m³ Gülle in den Behälter gepumpt. Die auf vorzugsweise 20° - 25° C aufgewärmte Gülle gelangt dann in die ringförmige Säuregärungskammer 4, in der die Vorgärung durch Säurebakterien stattfindet. Die dabei anfallenden Gase, im wesentlichen $CO_2$, $H_2$ und $H_2S$ können den Behälter über das Entlüftungsrohr 6 verlassen. Die Säuregärungskammer 4 ist über die zylindrische Wandung 15 vom Hauptgärraum 10 abgetrennt, wobei jedoch in Nähe des Behälterbodens die Wandung 15 endet, so daß sich eine zylindrische Öffnung 9 zum Hauptgärraum ergibt, die vorgegorene Gülle gelangt über die Öffnung 9 dann in den ebenfalls ringförmigen Hauptgärraum 10. Es ist eine pH-Meßvorrichtung 46 vorgesehen, mit der der pH-Wert der Gülle beim Verlassen der Säuregärungskammer gemessen werden kann. In diesem Hauptgärrum 10 findet der eigentliche Gärprozeß statt, in dem mit Hilfe von Methanbakterien das Biogas entwickelt wird. Das entstandene Biogas kann über eine Rohröffnung in Nähe der Behälterdecke und eine Abführleitung 16 entweichen, wobei die Biogasleitung aus Frostschutzgründen in vorteilhafter Weise durch den Behälter von oben nach unten hindurchgeführt wird. Die zylindrische Außenwandung des Hauptgärraums 10 ist mit 34 bezeichnet. Für den Auslauf der ausgegorenen Gülle ist ein Überlaufrohr 41 im Behälter vorgesehen, das sich im Hauptgärraum 10 neben der Wandung 15 zur Säuregärkammer 4 befindet. Die Höhe des Überlaufrohrs 41 bestimmt sich nach der maximal vorgesehenen Füllhöhe des Behälters. Das Überlaufrohr ist durch den Behälterboden durchgeführt und weist in dem Raum unter dem Behälter ein siphonartiges Knie 42 auf, durch das verhindert wird, daß das Biogas über den Überlauf den Behälter verläßt.

Im Säuregärraum 4 herrscht über der Gülle Atmosphärendurck, wohingegen im Hauptgärraum über der Gülle ein Überdruck von ca. 35 mbar herrscht, der ausreichend ist, daß das Biogas mit genügend hoher Geschwindigkeit über die Leitung 16 ausströmen kann.

Im oberen Bereich des Behälters ist eine Walze 8 gelagert, die zum Beispiel mittels eines Motors um ihre horizontale Achse 29 drehbar ist. Als Mischkörper sind für den Hauptgärraum zwei gleiche Betonplatten 11 vorgesehen, die mit Seilen 25, 26 bzw. 23, 24 an der Walze 8 aufgehängt sind, wobei sich bei Drehung der Walze 8 die Seile 25, 26 von der Walze abwickeln, während sich gleichzeitig die Seile 23, 24, an denen der andere Mischkörper hängt, aufwickeln, so daß sich der linke Mischkörper nach unten und der rechte Mischkörper nach oben bewegt bzw. umgekehrt. In der Behälterdecke 13 ist eine Mannluke 17 vorgesehen, so daß man bei Bedarf in den Behälter hineinschauen oder einsteigen kann. Für die Durchmischung in der inneren Säuregärkammer 4 ist vorzugsweise ein ringförmiger Mischkörper 5 vorgesehen, der als Schwimmkörper ausgebildet ist. Der Schwimmkörper kann zum Beispiel aus Polyester sein oder ein luftgefüllter Hohlkörper aus Gummi oder dergleichen. An dem Schwimmkörper 5 sind ebenfalls Seile 27, 28 befestigt, die sich auf der Walze 8 aufwickeln können, und die über untere Umlenkrollen 7 geführt sind. Werden nun die Seile 27, 28 bei Drehung der Walze von dieser abgewickelt, kann der Schwimmkörper 5 langsam aufschwimmen, und sorgt so für eine Durchmischung der Gülle im Säuregärraum 4, ohne daß dabei stärkere Strömungen entstehen, die die Bakterien beeinträchtigen. Durch die Aufhängung der Mischkörper ist es demnach möglich, bei Drehung der Walze sowohl die Mischkörper 11 als auch den Schwimmkörper 5 gleichzeitig zu bewegen und somit mit nur einem Antrieb die Gülle in beiden Gärräumen 4, 10 zu durchmischen.

Der ringförmige Mischkörper im Innenraum kann aber ebenfalls aus Beton oder dergleichen sein und an der Walze 8 mit Seilen direkt aufgehängt werden, so daß sie Umlenkrollen und die mit diesen verbundene Mechanik entfallen kann.

Durch die Ausbildung der Mischkörper wird quasi der gesamte Gärraum erfaßt. Man kann auch die Walze 8 anhalten und das Gärgut nur schichtweise in verschiedenen Höhen bewegen. Da der Grad der Vergärung des Gärguts im Behälter von unten nach oben fortschreitet, können die Ausfaulphasen erhalten bleiben. Bei der Durchmischung in vertikaler Richtung wird die Ausbildung einer Schwimmdecke und eines eventuellen Bodensatzes im Behälter verhindert.

Da das frische Gärgut schnell mit den Methanbakterien des angefaulten Gärgutes in Kontakt kommt, wird der Beginn des Gärprozesses beschleunigt. Das erfindungsgemäße Verfahren hat somit gegenüber bekannten Verfahren den zusätzlichen Vorteil, daß eine Impfung des Gärmediums mit einem Bakterienkonzentrat enfallen kann. Da die Gärung ohne Totzeit sofort in Gang kommt, kann eine sehr kurze Verweildauer des Gärguts in der Hauptgärkammer erzielt werden, die bei etwa 2 bis 3 Tagen liegt. Da die ausgegorene Gülle weitgehend durch Frischgülle verdrängt wird, geht kein Gärraum verloren. Durch den Wärmetauscher 3 ist die Temperatur in der Säuregärkammer 4 praktisch konstant. Da die Säurebakterien optimale Lebensbedingungen vorfinden, können sie den Sauerstoffgehalt der Gülle auf ein Minimum reduzieren. Dies wird auch durch die gleichmäßige Durchmischung mit Hilfe des Schwimmkörpers 5 in schonender Weise unterstützt. Durch den geringen Sauerstoffgehalt der Gülle beim Eintreten in den Hauptgärraum 10 wird dort der Gärprozeß beschleunigt und somit die Verweildauer der Gülle verringert. Da durch die behutsame Durchmischung der Gülle im Hauptgärraum 10 keine für die Methanbakterien störenden Strömungen und Turbulenzen auftreten, werden auch deren Lebensbedingungen begünstigt. Die Ausbeute an Biogas wird somit erhöht. Die erfindungsgemäße Anlage ist auch für Gülle mit einem höheren Anteil an unzerkleinerten Feststoffen sowie für alle vergärbaren dickflüssigen Medien geeignet.

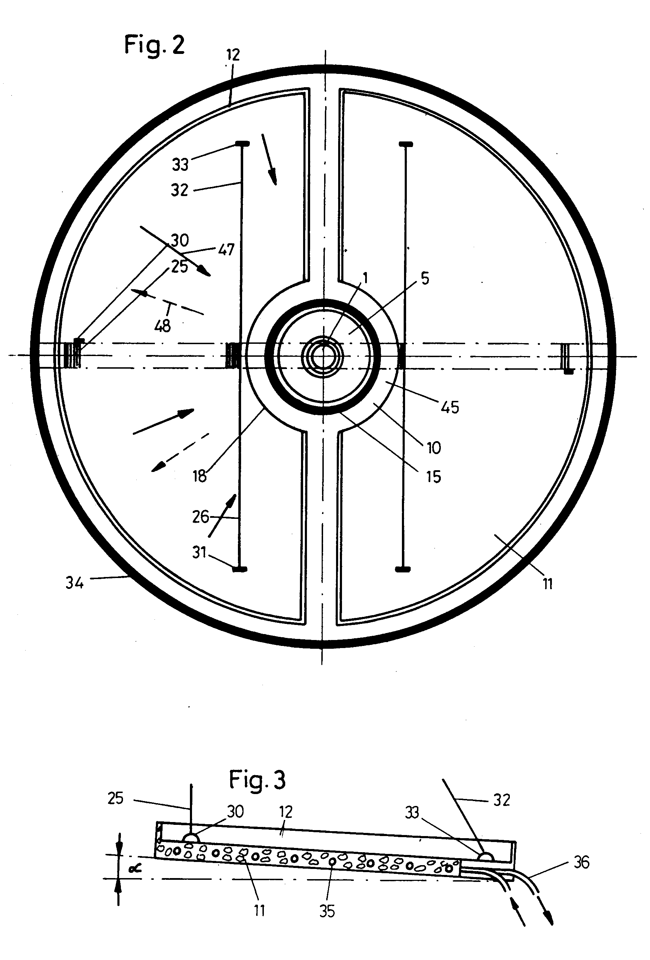

Wie Fig. 2 zeigt, sind zwei gleiche Mischkörper 11 vorgesehen, deren Umriß einem Halbkreissektor entspricht, wobei wegen der Säuregärkammer 4 innen jeweils eine halbkreissektorförmige Ausnehmung ist. Die beiden Mischkörper 11 erfassen so nahezu den gesamten Behälterquerschnitt bis auf die verbleibenden Randzonen, an denen die Gülle bei Bewegung der Mischkörper vorbeiströmt. Die Mischkörper sind jeweils an drei Seilen 25, 26, 32 aufgehängt, wobei die Aufhängepunkte 30, 31, 33 z.B. auf einem Kreisbogen angeordnet sein können, dessen Mittelpunkt die Behälterachse ist. Einer der Aufhängepunkte 30 liegt dabei nahezu unterhalb der Walze, so daß das Seil 25 etwa senkrecht hängt, während die anderen beiden Seile 26, 32 schräg geführt sind. Ein gleichmäßiges Aufwickeln der drei Seile auf die Walze und somit einen reibungslosen Bewegungsablauf kann man wegen der unterschiedlichen Länge der Seile 25 einerseits und 26 und 32 andererseits dadurch erreichen, daß man die Dicke der Walze variiert.

Um die Strömungsverhältnisse im Behälter zu optimieren, wie weiter unten erläutert wird, weisen die Mischkörper eine umlaufende vertikale Aufkantung 12, zum Beispiel in Form eines Blechs, auf, wobei jedoch jeweils der mittlere kreisbogenförmige Bereich 18 keine Aufkantung hat.

Fig. 3 zeigt einen Mischkörper 11 für den Hauptgärraum, der als Betonplatte ausgebildet ist. Die Betonplatte ist dabei mit den Seilen 25, 32 vorzugsweise so im Behälter aufgehängt, daß die Platte einen Neigungswinkel $\alpha$ von wenigen Grad zur Horizontalen aufweist. In der Betonplatte sind als Heizvorrichtung Warmwasserrohre 35, zum Beispiel nach Art einer Fußbodenheizung mäanderförmig verlegt. Die Zu- und Ableitung des Warmwassers geschieht mittels der Schläuche 36. Die Seile der Aufhängevorrichtung 25, 32 sind über Anker 30, 33, die zum Beispiel in Beton eingegossen sind, an dem Mischkörper 11 befestigt. Am Außenrand weist die Mischkörperplatte 11 eine umlaufende Aufkantung 12 aus Beton in Form eines Blechs auf.

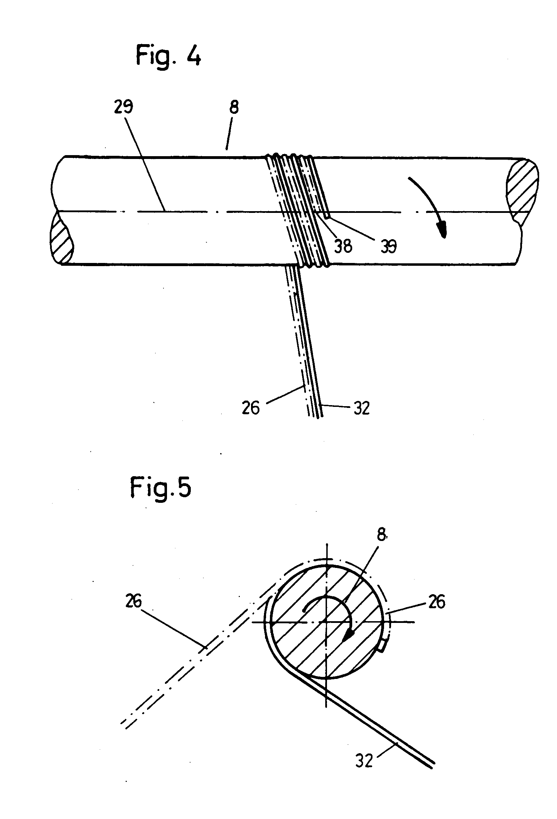

Fig. 4 zeigt eine vergrößerte Darstellung der Walze 8, auf der die Halteseile für die Mischkörper aufgewickelt werden. Die Walze 8 dreht sich um ihre horizontale Achse 29. Die Seile sind durch geeignete Befestigungsmittel an ihren Endpunkten 38, 39 auf der Walze befestigt. Damit ein reibungsloses Aufwickeln der Seile 26, 32 auf die Walze gewährleistet ist, ist es besser, wenn sich die Seile so aufwickeln, daß in Richtung der Walzenachse jeweils abwechselnd einer Wicklung des Seiles 26 eine Wicklung des Seiles 32 folgt. Die Seile 26, 32 wickeln dann sauber nebeneinander und nicht übereinander auf, was zu einem Verklemmen der Seile führen würde. Dabei ist es so, daß das Seil 32, das sich bei Drehung der Walze im Uhrzeigersinn gemäß Fig. 5 zuerst auf die Walze aufwickelt, eine Führung für das andere Seil 26 bildet, so daß ein sauberes Aufwickeln der Seile jeweils nebeneinander erfolgt.

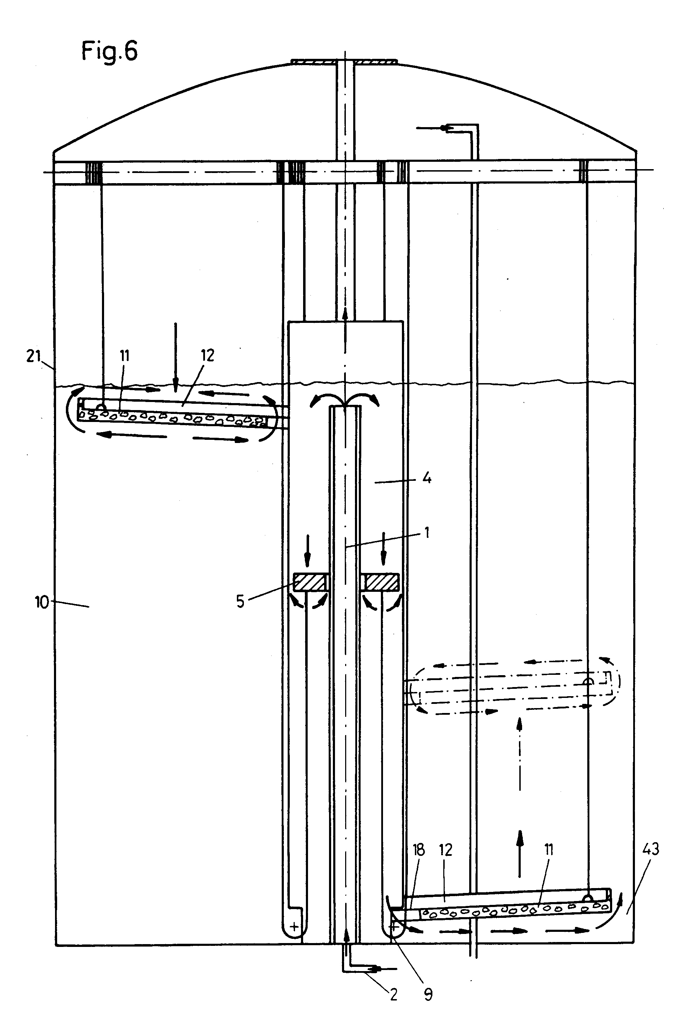

Im folgenden wird auf Fig. 6 Bezug genommen, die Strömungsverhältnisse im Behälter bei Bewegung der Mischkörper noch einmal verdeutlicht. Im inneren Säuregärraum 4 strömt die Gülle bei Bewegung des Schwimmkörpers 5 in Pfeilrichtung nach unten, in einer Aufwärtsbewegung seitlich am Schwimmkörper 5 vorbei und somit der frisch am oberen Ende der Aufwärmzone 1 in die Säuregärkammer 4 gelangenden Gülle entgegen. Da ständig frische Gülle nachgepumpt wird, strömt die Gülle am unteren Ende der Säuregärkammer durch die Öffnung 9 in den Hauptgärraum 10. Es ist nun wichtig, daß verhindert wird, daß die Gülle von dort entlang der Wandung zwischen den beiden Gärkammern auf dem kürzesten Weg nach oben strömt und so in das Überlaufrohr 41 gelangt. Die Verweildauer dieser Gülle im Behälter wäre dann für eine ausreichende Vergärung zu kurz. Deshalb haben die Mischkörper 11 an den Rändern die Aufkantung 12, die jedoch im Bereich des inneren Kreisbogens nicht besteht, so daß in diesem Bereich 18 die Mischkörperplatte flach ist. Die durch die Öffnung 9 in den Hauptgärraum 10 einströmende Gülle strömt, wenn sich die Mischkörperplatte 11 in Höhe der Öffnung befindet, unter der Platte durch, im Behälter radial nach außen und dort an dem Mischkörper vorbei. Die einströmende Frischgülle erreicht so in kurzer Zeit die äußeren Randzonen des Behälters 43, in denen sich Alt-Gülle befindet und verdrängt diese. Durch die Aufkantung 12 am Rand des Mischkörpers tritt die umgekehrte Strömung von außen über den Rand der Aufkantung nach innen, bei der der Strömungswiderstand wesentlich höher wäre, nicht auf. Die besondere Ausbildung der Mischkörper sorgt demnach für eine praktisch 100 %ige Ausfaulrate des Gärguts. Außerdem verhindert die Erwärmung der Gülle über die Mischkörper, die mit der Gülle in allen Bereichen des Behälters in Kontakt gelangen, die Ausbildung von Kälte- oder Wärmenestern.

Bei der Bewegung der Mischkörper nach unten (siehe linker Mischkörper in der Darstellung gemäß Fig. 6) strömt die Gülle an beiden Seiten des Mischkörpers, also innen und außen, vorbei und under der Platte radial von der Plattenmitte nach innen und außen aber über der Platte von außen und innen radial zur Plattenmitte. Bei der Aufwärtsbewegung der Mischkörper (rechter Mischkörper in der Darstellung gemäß Fig. 6) strömt die Gülle unter der Platte von innen nach außen (siehe auch Pfeile 48 in Fig. 2) und über der Platte von der Aufkantung radial nach innen (siehe auch Pfeile 47 in Fig. 2). Da der Mischkörper jedoch eine leichte Neigung zur Behältermitte hin aufweist, wird über der Platte die Strömung von außen nach innen noch verstärkt. Hierdurch wird also der vorbeschriebene Effekt, der die gleichmäßige Durchmischung von Frischgülle mit der in den Außenzonen befindlichen Alt-Gülle bewirkt, unterstützt.

Die Durchmischung der Gülle durch Bewegung der plattenförmigen Mischkörper 11 in vertikaler Richtung hat folgenden weiteren Vorteil. Wenn die Bakterien ihr Stoffwechselprodukt Methan entwickeln, bilden sich Millionen sehr kleiner Bläschen. Wegen des Feststoffanteils in der Gülle können diese winzigen Bläschen mit ihrem geringen Auftrieb nicht schnell genug im Behälter nach oben aufsteigen. Es besteht dadurch die Gefahr, daß sich das Stoffwechselprodukt der Bakterien im gesamten Gärbereich anreichert. Dadurch werden die Lebensbedingungen der Bakterien verschlechtert, es kann zur Absterbung der Bakterien führen. Somit ist es erwünscht, das Stoffwechselprodukt so schnell wie möglich abzuführen. Da die plattenförmigen Mischkörper eine große Oberfläche aufweisen und bei Bewegung der Mischkörper die winzigen Bläschen mit den angewärmten Platten in Kontakt gelangen, findet an den Plattenflächen eine Vereinigung kleiner Bläschen zu größeren Bläschen statt. Die größeren Bläschen haben eine größere Auftriebskraft, die das Aufsteigen erleichtert und die Entleerung des Behälters mit dem Stoffwechselprodukt der Bakterien, das heißt dem Biogas, wird so beschleunigt. Die im Behälter von unten nach oben zunehmende Größe der Bläschen ist in der Darstellung gemäß Fig. 1 angedeutet.

Durch die Geschwindigkeit der Vertikalbewegung der Mischkörper kann die Dicke der jeweils oberhalb bzw. unterhalb der Mischkörper durchmischten Gülleschicht bestimmt werden. Bei langsamer Bewegung der Mischkörper wird nur eine Schicht geringer Dicke (Höhe) oberhalb und unterhalb der Mischkörperplatten bewegt und durchmischt. Bei schnellerer Bewegung nimmt diese Dicke der bewegten Schicht zu. Durch diese quasi schichtweise Durchmischung bleiben die Gülleschichten mit je nach Abstand vom Behälterboden verschiedenen Fäulnisphasen jeweils schichtweise erhalten.

Bei der in den Figuren 1 und 6 dargestellten Variante der erfindungsgemäßen Anlage wird von einem stehenden im wesentlichen kreiszylindrischen Behälter ausgegangen. Der Behälter kann jedoch ebensogut einen rechteckigen oder vieleckigen Querschnitt aufweisen. Der erfindungsgemäße Behälter muß auch nicht stehend ausgebildet sein, sondern kann vielmehr auch, ähnlich zum Beispiel wie ein Heizöltank, liegend ausgeführt werden. Bei einem solchen liegenden Gärbehälter werden die Mischkörper zur Durchmischung der Gülle ebenfalls als Platten ausgebildet und im Behälter langsam in achsialer Richtung bewegt. In diesem Fall ist es nicht möglich, die Mischkörper an Seilen im Behälter aufzuhängen. Statt dessen können die Mischkörper jedoch mit Hilfe von im Behälter angeordneten Schienen und geeigneten Antriebsmitteln im Behälter bewegt werden. Die Mischkörper werden dann in den Schienen geführt und durch die Antriebsmittel in axialer Richtung verschoben. Die Durchmischung erfolgt dann in analoger Weise, wie bei dem in den Figuren 1 und 6 dargestellten Behälter, so daß auch bei einer solchen Anlage die oben beschriebenen Vorteile der Durchmischung gegeben sind.

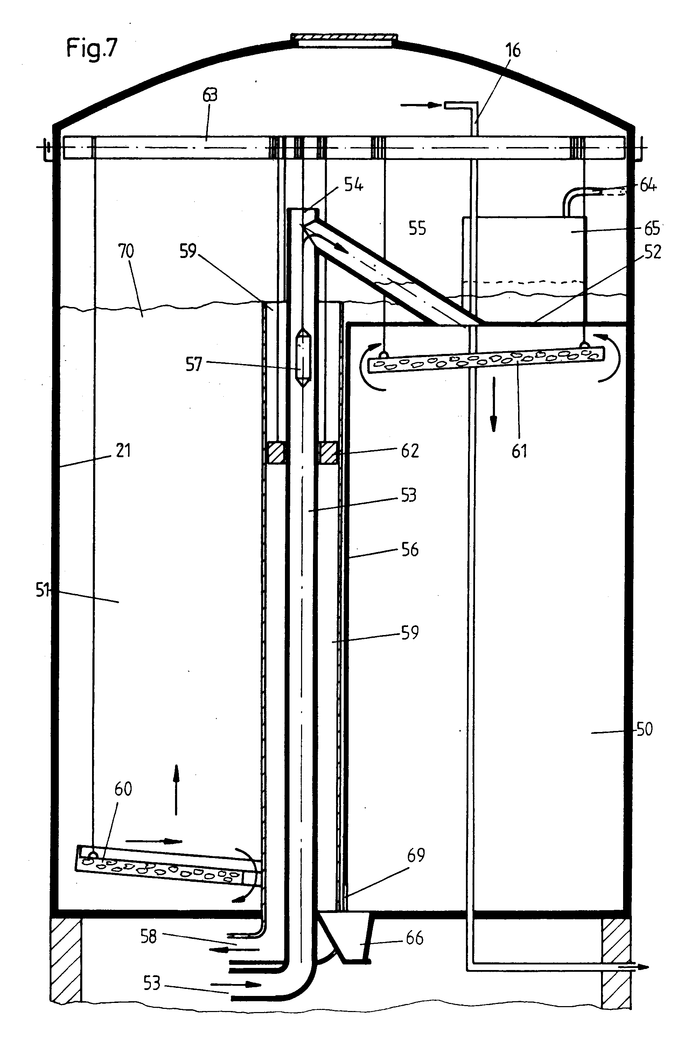

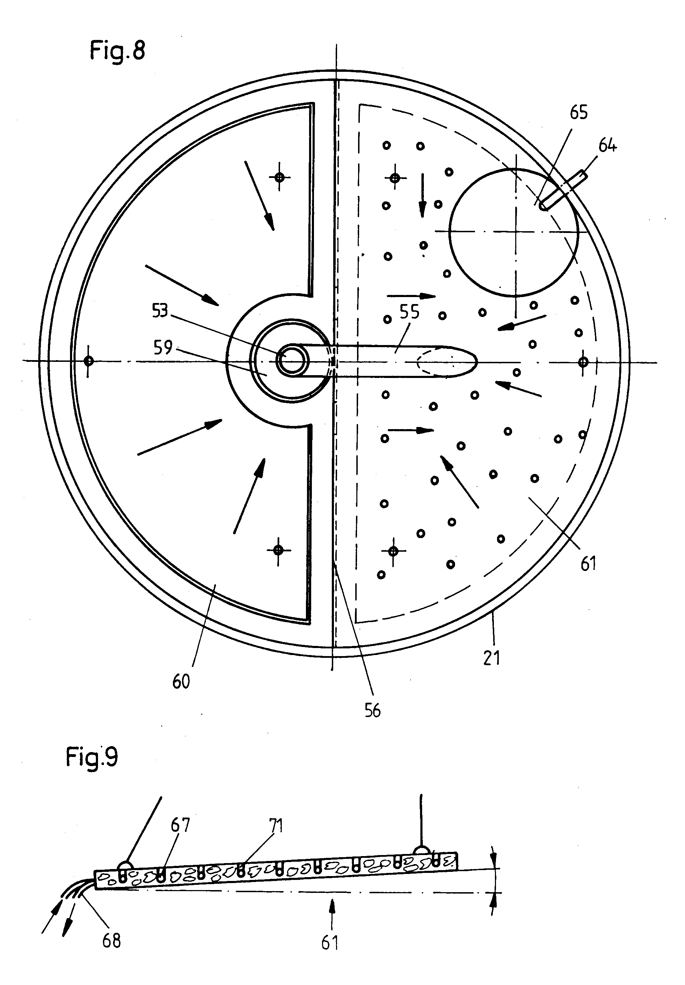

Im folgenden wird anhand der Figuren 7 bis 9 eine Variante des erfindungsgemäßen Verfahrens und der erfindungsgemäßen Anlage näher beschrieben. Bei dieser Ausführungsform der Anlage besteht der Behälter 21 aus der Säuregärkammer 50 und der Hauptgärkammer 51, die durch eine Trennwand 56, die quer durch den Behälter verläuft, voneinander getrennt sind. Die Säuregärkammer 50 ist oben durch eine Verschlußdecke 52 verschlossen. An diese Verschlußdecke schließt sich seitlich eine Glocke 65 an, deren obere Wandung höher liegt, als die Verschlußdecke 52. Diese Glocke dient im wesentlichen als Schaumfalle. Die in der Säuregärkammer 50 entstehenden nicht brauchbaren Gase, nämlich im hauptsächlichen $CO_2$, $H_2$ und $H_2S$, können in der Kammer nach oben hin über die Glocke 65 und ein Gasableitrohr 64 aus dem Behälter 21 entweichen. Bei dieser Anlage wird die Gülle zunächst über das Zuleitungsrohr 53 in den Behälter gepumpt. Das Zuleitungsrohr 53 ist etwas seitlich von der Mitte des Behälters angeordnet. Die eingepumpte Gülle steigt in diesem Rohr nach oben, bis sie die Abzweigung zu dem Rohr 55 erreicht hat und fließt dann über das Rohr 55 in die Säuregärkammer 50. Aus Sicherheitsgründen ist am oberen Ende des Zuleitungsrohrs 53 eine Entlüftung 54 für die in dem Zuleitungsrohr 53 sich entwickelnden Gase vorgesehen. Diese Gase steigen nach oben und können am oberen Ende 54 des Rohrs entweichen. Falls es erforderlich ist, wird der Gärbehälter mit einem Überdruckventil versehen. Auch für das Zuleitungsrohr 53 ist bereits ein Mischkörper 57 vorgesehen, der ebenfalls an der Walze 63 im Behälter aufgehängt wird. Auch dieser Mischkörper ist vorzugsweise aus Beton. Der Mischkörper ist wegen der Enge des Rohrs etwa zylindrisch ausgebildet und hat eine kegelige Spitze, das heißt insgesamt etwa die Form einer Granate. Das Eigengewicht des Mischkörpers 57 reicht, wenn dieser aus Beton ausgebildet ist, in der Regel aus, um bei dessen Absenken die Gülle im Zuleitungsrohr 53 zu durchmischen, auch wenn diese Festkörperanteile enthält. Durch die Durchmischung der Gülle im Zuleitungsrohr wird außerdem im Rohr ein Temperaturausgleich erzielt und ein Temperaturprofil von innen nach außen verhindert. Dadurch ist der Wärmeaustausch zwischen der kühleren Frischgülle im Zuleitungsrohr und der wärmeren Altgülle im Ablaufrohr 59 optimiert. In der Säuregärkammer findet dann die Säuregärung statt. Wenn diese Säuregärung aerob verlaufen soll, wird die Gülle in der Säuregärkammer 50 über den dort aufgehängten plattenförmigen Mischkörper 61 belüftet. In diesem Mischkörper 61 sind Rohre 67 verlegtmit Verbindungsstücken, die Öffnungen zur Oberfläche des Mischkörpers hin aufweisen. Dabei ist es vorteilhaft, wenn Mittel vorgesehen sind, die Luftauslaßöffnungen an der Mischkörperoberfläche zu verschließen, damit keine Gülle in die Luftleitungen eindringen kann. Die Versorgung der Rohrleitungen 67 im Mischkörper mit Luft oder Sauerstoff geschieht über mit diesem verbundene aus dem Mischkörper 61 herausgeführte Schleppschläuche 68. Solche Schleppschläuche sind im Prinzip von zum Beispiel Hydraulikschläuchen bei Aufzügen her bekannt. Die Schleppschläuche 68 werden deshalb verwendet, da ja die Mischkörperplatte 61 im Behälter mittels der Seile auf- und abbewegt wird und bei dieser Bewegung die Schläuche nicht hinderlich sein dürfen. Nach der Säuregärung kann die Gülle die Säuregärkammer 50 über eine Öffnung in der Trennwand 56 in Nähe des Behälterbodens verlassen. Diese Verbindungsöffnung 69 ist dabei relativ klein, zum Beispiel bei rechteckiger Ausführung etwa 20 cm x 30 cm, damit keine ungewollte Durchmischung der Gülle aus der Säuregärungskammer 50 mit der Gülle aus der Hauptgärkammer 51 eintritt. Es können auch zwei solche Verbindungsöffnungen vorgesehen sein, die rechts und links des Zuleitungsrohrs 53 liegen. Die Gülle gelangt somit in die Hauptgärkammer 51 in Nähe des Bodens, wo die eigentliche Methangärung beginnt. Das sich entwickelnde Biogas steigt in der Hauptgärkammer 51 nach oben und wird wiederum durch ein Methanableitrohr 16 aufgefangen und aus dem Behälter abgeführt. Das Methanableitrohr kann wie bei der Ausführungsform nach Fig. 1 durch die Gülle hindurchgeführt werden, damit es nicht einfriert. Der Flüssigkeitspegel 70 in der Hauptgärkammer kann so hoch liegen, daß die Verschlußdecke 52 der Säuregärkammer 50 bedeckt wird. Die Höhe des Flüssigkeitspegels in der Hauptgärkammer 51 wird durch ein Ablaufrohr 59 für die ausgegorene Gülle der Hauptgärkammer bestimmt. Dieses Ablaufrohr 59 ist als Überlaufrohr ausgebildet, so daß die ausgegorene Gülle der Hauptgärkammer oben in dieses Ablaufrohr einläuft. Das Ablaufrohr 59 ist dabei konzentrisch um das Einlaufrohr 53 für die Gülle, das zur Säuregärkammer 50 führt, angeordnet. Die Gülle läuft dann über den Auslauf 58. Die konzentrische Anordnung des Ablaufrohrs um das Einlaufrohr hat den Vorteil, daß ein direkter Wärmeaustausch zwischen der kühleren Frischgülle und der warmen verbrauchten Gülle über die Wandung des Einlaufrohrs 53 stattfindet. Es hat sich jedoch gezeigt, daß wegen der dickflüssigen Konsistenz der Gülle, die auch Festkörperanteile enthält, in dieser keine thermische Zirkulation und somit kein ausreichender Wärmeausgleich von außen nach innen stattfindet, so daß die Erwärmung über die Rohrwandung des Einlaufrohrs 53 allein nicht genügt. Damit der Wärmetausch zwischen der Alt-Gülle und der Frischgülle über die Rohrwandung des Rohrs 53 effektiv ist, muß daher unbedingt eine Durchmischung der Alt- Gülle im Ablaufrohr 59 stattfinden. Hierzu ist ein oder auch mehrere Betonmischkörper 62 für die Durchmischung der Gülle in dem Ringraum des Ablaufrohrs 59 vorgesehen. Die Frischgülle gelangt also bereits vorgewärmt über das Zuleitungsrohr 53 in die Säuregärkammer 50, wobei für diese Vorwärmung die bei den Fermentationsprozessen entstehende Wärme ausgenutzt werden kann. Auch der Betonmischkörper 62 ist über ein Seil an der Walze 63 aufgehängt und wird im Behälter auf- und abbewegt. Vorzugsweise ist im Gärbehälter 21 im Boden im Bereich der Verbindungsöffnungen 69 in der Trennwand zwischen den beiden Gärkammern noch ein Sandabschneider 66 angeordnet, der die abgeschiedenen Schlamm- und Sandanteile aus der Gülle auffängt. Die Strömungsverhältnisse bei dem Behälter entsprechend der Ausführungsform gemäß den Figuren 7 und 8 sind ähnlich wie bei der Anlage gemäß Fig. 1 und 6. In der Darstellung gemäß Fig. 7 und 8 ist jedoch nur die Mischkörperplatte 60 in der Hauptgärung mit der umlaufenden Aufkantung versehen, wohingegen die Mischkörperplatte 61 in der Säuregärkammer 50 keine Aufkantung aufweist. Die Gülle bewegt sich somit bei Bewegung der Mischkörper entsprechend den Pfeilen in der Darstellung gemäß Fig. 8. In der Säuregärung erfolgt die Strömung bei dem Mischkörper 61 ohne Aufkantung jeweils von der Mitte der Platte zum Plattenrand hin. Die dargestellten Strömungsverhältnisse haben sich für eine aerobe Säuregärung als besonders günstig erwiesen. Gemäß der Erfindung besteht also auch die Möglichkeit, je nach Verwendung von Mischkörpern mit oder ohne Aufkantung 12 die Strömungsverhältnisse im Behälter unter und über den Mischkörperplatten zu variieren und somit die Durchmischung zu optimieren.

Die Darstellung gemäß Fig. 9 zeigt einen Schnitt durch den Mischkörper 61, der für die Säuregärkammer 50 gemäß Fig. 7 vorgesehen ist. Die Einleitung bzw. Ableitung der Luft erfolgt über die an den Mischkörper angeschlossenen Schleppschläuche 68. Die Schläuche 68 stehen in Verbindung mit Rohren 67, durch die Luft geleitet wird, die im Mischkörper verlegt sind. Damit die Belüftung der Gülle an der Oberseite bzw. Unterseite des Mischkörpers erfolgen kann, zweigen von den Rohren 67 Verbindungsstücke 71 mit Öffnung zur Ober- bzw. Unterseite der Mischkörperplatte ab. Wenn man verhindern will, daß, wenn keine Lufteinleitung erfolgt, Gülle in das System von Rohren 67 einfließen kann, kann man die Mischkörperplatte 61 mit einer Siebfolie bespannen. Diese Siebfolie hat sehr feine Öffnungen von zum Beispiel 10 - 15 $\mu$m, so daß durch diese keine Gülle eindringen kann.

**Bezugszeichenliste**

1. Aufwärmzone
2. Zuleitung für Gülle
3. Wärmetauscherrohr (doppelwandig)
4. Säuregärungsraum
5. Schwimmkörper
6. $CO_2/H_2S$- Entlüftungsrohr
7. Umlenkrolle
8. Walze
9. Öffnung zum Hauptgärraum
10. Hauptgärraum
11. Betonplatten zur Vermischung des Mediums
12. Aufkantung
13. Behälterdecke
14. Hohlraum unter dem Behälter
15. Wandung
16. Methan-Abführleitung
17. Mannluke
18. flacher Bereich ohne Aufkantung
19. Füllhöhe
20. Fundament
21. Behälter
22. Pumpe
23. Zugseile für Betonplatten
24. Zugseile für Betonplatten
25. Zugseile für Betonplatten
26. Zugseile für Betonplatten
27. Zugseile für Schwimmkörper
28. Zugseile für Schwimmkörper
29. Achse der Walze
30. Befestigungspunkt für Zugseil
31. Befestigungspunkt für Zugseil
32. Zugseil
33. Befestigungspunkt für Zugseil
34. Behälterwandung
35. Heizschlangen (mäanderförmig) $\alpha$ Winkel zur Waagrechten
36. Schläuche für Warmwasser
37. Schläuche für Warmwasser
38. Befestigungspunkte für Seile auf der Walze
39. Befestigungspunkte für Seile auf der Walze
40. Biogas-Leitung
41. Auslauf für ausgefaulte Gülle
42. Knie als Gasfalle
43. äußere Zone des Behälters
44. Gasbläschen
46. pH-Meßgerät
47. Pfeile
48. Pfeile
50. Säuregärungskammer
51. Hauptgärkammer
52. Verschlußdecke
53. Zuleitung (Gülle)
54. Entlüftung
55. Verbindungsrohr
56. Trennwand

57. Mischkörper
58. Gülle-Ableitung
59. Gülle-Ablaufrohr
60. Mischplatte
61. Mischkörper
62. Mischkörper
63. Walze
64. Gasableitrohr
65. Glocke
66. Schlammabscheider
67. Sauerstoffleitungen
68. Schleppschläuche
69. Verbindungsöffnung

**Patentansprüche**

1. Verfahren zur Herstellung von Biogas durch Vergärung dickflüssiger Medien, bei dem die Vergärung in einem Gärbehälter in zwei Stufen erfolgt, einer ersten Säuregärungsstufe, bei der mit Hilfe von Säurebakterien Sauerstoff verbraucht wird, und einer anschließenden zweiten Hauptgärungsstufe, bei der das Biogas erzeugt wird, dadurch gekennzeichnet, daß in beiden Gärungsstufen die Durchmischung des Gärmediums durch langsame kontinuierliche Bewegung von etwa plattenförmigen Mischkörpern (11, 5) in axialer Richtung des Gärbehälters erfolgt.

2. Verfahren nach Anspruch 1, dadurch gekennzeichnet, daß die Säuregärung aerob erfolgt.

3. Verfahren nach Anspruch 1, dadurch gekennzeichnet, daß die Säuregärung anaerob erfolgt.

4. Verfahren nach Anspruch 1, dadurch gekennzeichnet, daß die ausschließliche oder eine zusätzliche Aufwärmung des Gärmediums durch die Mischkörper erfolgt.

5. Verfahren nach Anspruch 3 oder 4, dadurch gekennzeichnet, daß das Gärmedium vor Eintritt in die Säuregärungsstufe mittels eines Wärmetauschers vorgewärmt wird.

6. Verfahren nach Anspruch 2, dadurch gekennzeichnet, daß eine Kühlung des Gärmediums durch Wärmeabfuhr über die Mischkörper erfolgt.

7. Verfahren nach Anspruch 2 oder 6, dadurch gekennzeichnet, daß über die Mischkörper Sauerstoff in das Gärmedium eingebracht wird.

8. Verfahren nach einem der Ansprüche 1 bis 7, dadurch gekennzeichnet, daß die Zufuhr des Gärmediums in den Gärbehälter durch eine pneumatische Fördereinrichtung erfolgt.

9. Verfahren nach einem der Ansprüche 1 bis 8, dadurch gekennzeichnet, daß durch die Änderung der Geschwindigkeit der Axialbewegung der Mischkörper (11, 5) die Dicke der an die Mischkörper angrenzenden durchmischten Schicht variiert wird.

10. Verfahren nach einem der Ansprüche 1 bis 9, dadurch gekennzeichnet, daß der pH-Wert des Gärmediums beim Verlassen der Säuregärungsstufe gemessen wird.

11. Verfahren nach einem der Ansprüche 1 bis 10, dadurch gekennzeichnet, daß das Gärmedium Gülle oder Klärschlamm ist.

12. Anlage zur Herstellung von Biogas durch Vergärung dickflüssiger Medien mit einem vorzugsweise zylindrischen Behälter, in dem das Gärmedium vergoren wird, dadurch gekennzeichnet, daß die Durchmischung durch etwa plattenförmige Mischkörper (11) erfolgt, die im Behälter (21) in axialer Richtung bewegt werden.

13. Anlage nach Anspruch 12, dadurch gekennzeichnet, daß die plattenförmigen Mischkörper (11) in einem stehenden Behälter aufgehängt und in vertikaler Richtung anhebbar bzw. absenkbar sind.

14. Anlage nach Anspruch 12 oder 13, dadurch gekennzeichnet, daß die plattenförmigen Mischkörper bei ihrer Axialbewegung den überwiegenden Teil des Behälterquerschnitts erfassen, wobei aber zwischen Mischkörpern und Behälterwandung ein ausreichender Freiraum verbleibt, durch den das Gärmedium hindurchströmen kann.

15. Anlage gemäß Anspruch 14, dadurch gekennzeichnet, daß die Mischkörper an Seilen aufgehängt sind und das Anheben und Absenken der Mischkörper durch Aufwickeln bzw. Abwickeln der Seile auf eine um ihre horizontale Achse drehbare Walze (8) erfolgt.

16. Anlage nach einem der Ansprüche 12 bis 15, dadurch gekennzeichnet, daß die Mischkörper (11, 60, 61) zur Behältermittelachse hin etwas geneigt sind.

17. Anlage nach einem der Ansprüche 12 bis 16, dadurch gekennzeichnet, daß die Mischkörper (11, 60) eine umlaufende Aufkantung (12) am Plattenrand aufweisen, mit Ausnahme eines inneren kreisbogenförmigen Bereichs (19) ohne Aufkantung.

18. Anlage nach einem der Ansprüche 12 bis 17, dadurch gekennzeichnet, daß zwei etwa gleiche Mischkörper (11, 60, 61) im Behälter vorgesehen sind, die mit Seilen und der Walze (8) so aufgehängt sind, daß bei Drehung der Walze sich die Mischkörper jeweils gegenläufig in vertikaler Richtung bewegen.

19. Anlage nach einem der Ansprüche 12 bis 18, dadurch gekennzeichnet, daß die Mischkörper (11, 60,61) jeweils an drei Punkten im Behälter aufgehängt sind.

20. Anlage nach einem der Ansprüche 12 bis 19, dadurch gekennzeichnet, daß die Mischkörper (11, 60, 61) aufheizbar bzw. kühlbar sind.

21. Anlage nach einem der Ansprüche 12 bis 20, dadurch gekennzeichnet, daß für die Aufheizung bzw. Kühlung der Mischkörper (11, 60, 61) in diesen Wasserrohre (35) verlegt sind.

22. Anlage nach einem der Ansprüche 12 bis 21, dadurch gekennzeichnet, daß in wenigstens einem der Mischkörper (61) Sauerstoffleitungen (67) verlegt sind, die mit Öffnungen in dem Mischkörper in Verbindung stehen, so daß das Gärmedium über den Mischkörper belüftet werden kann.

23. Anlage nach einem der Ansprüche 12 bis 22, dadurch gekennzeichnet, daß für die Sauerstoffversorgung des Mischkörpers (61) Schleppschläuche (68) vorgesehen sind.

24. Anlage nach einem der Ansprüche 12 bis 23, dadurch gekennzeichnet, daß für die Wasserversorgung der Mischkörper (11, 60, 61) Schleppschläuche vorgesehen sind.

25. Anlage nach einem der Ansprüche 12 bis 24, dadurch gekennzeichnet, daß die Mischkörper (11, 60, 61) einen halbkreisringförmigen oder halbkreisförmigen Umriß aufweisen.

26. Anlage nach einem der Ansprüche 12 bis 25, dadurch gekennzeichnet, daß die Mischkörper (11, 60, 61) aus Beton sind.

27. Anlage nach einem der Ansprüche 12 bis 26, dadurch gekennzeichnet, daß der Behälter (21) eine Sauregärungskammer (4) mit einem Gasentlüftungsrohr (6) zur Ableitung nicht brauchbarer Gase aufweise, die durch eine Öffnung (9) in Nähe des Behälterbodens mit einem Hauptgärraum (10) verbunden ist, der in seinem oberen Bereich die Abführleitung (16) für das Biogas aufweist.

28. Anlage nach einem der Ansprüche 12 bis 27, dadurch gekennzeichnet, daß die Säuregärungskammer (4) ringförmig ist und innen in Nähe der Behälterachse angeordnet ist, wobei der Hauptgärraum (10) diese konzentrisch umgibt.

29. Anlage nach einem der Ansprüche 12 bis 28, dadurch gekennzeichnet, daß der Behälter durch eine vertikale Trennwand in zwei Kammern aufgeteilt ist, von denen die eine als Säuregärungskammer (50) und die andere als Hauptgärkammer (51) dient, wobei die Säuregärungskammer (50) nach oben hin durch eine Verschlußdecke (52) abgeschlossen ist.

30. Anlage nach einem der Ansprüche 12 bis 29, dadurch gekennzeichnet, daß Säuregärungskammer (50) und Hauptgärkammer (51) in etwa gleiche Volumina aufweisen.

31. Anlage nach einem der Ansprüche 12 bis 30, dadurch gekennzeichnet, daß die Zufuhrleitung (2) für das Gärmedium im Behälterboden in eine innere Aufwärmzone (1) mündet, deren zylindrische Wandung von einem doppelwandigen Wärmetauscher (3) gebildet ist.

32. Anlage nach einem der Ansprüche 12 bis 31, dadurch gekennzeichnet, daß das Zufuhrrohr (53) für die Zufuhr des Gärmediums zur Säuregärungskammer (50) konzentrisch von dem Auslaufrohr (59) für den Auslauf des verbrauchten Gärmediums aus der Hauptgärkammer umgeben ist.

33. Anlage nach einem der Ansprüche 12 bis 32, dadurch gekennzeichnet, daß die Zufuhr des Gärmediums über eine pneumatische Fördereinrichtung erfolgt.

34. Anlage nach einem der Ansprüche 12 bis 33, dadurch gekennzeichnet, daß die pneumatische Fördereinrichtung (22) das Gärmedium mit Hilfe von mittels eines Kompressors erzeugter Druckluft schubweise in den Behälter pumpt.

35. Anlage nach einem der Ansprüche 12 bis 34, dadurch gekennzeichnet, daß die Biogasleitung (40) durch den Behälter (21) hindurchgeführt ist.

36. Anlage nach einem der Ansprüche 12 bis 35, dadurch gekennzeichnet, daß für die Durchmischung des Gärmediums in dem Ringraum des Auslaufrohrs (59) ein weiterer Mischkörper (62) vorgesehen ist, der ebenfalls in axialer Richtung bewegt wird.

37. Anlage nach einem der Ansprüche 12 bis 36, dadurch gekennzeichnet, daß für das Zufuhrrohr (53) ein weiterer etwa zylinderförmiger Mischkörper (57) mit kegeliger Spitze vorgesehen ist.

38. Anlage nach einem der Ansprüche 12 bis 37, dadurch gekennzeichnet, daß die Mischkörper (62, 57) ebenfalls an Seilen aufgehängt sind und mit Hilfe der Walze (63) in vertikaler Richtung bewegt werden.

39. Anlage nach einem der Ansprüche 12 bis 38, dadurch gekennzeichnet, daß der Mischkörper für die Durchmischung des Gärmediums in der Säuregärungskammer (4) ein Schwimmkörper (5) ist, an dem Seile (27, 28) befestigt sind, die über untere Umlenkrollen (7) umgelenkt und auf der Walze (8) aufgewickelt werden, so daß beim Abwickeln der Seile der Schwimmkörper aufschwimmen kann.

40. Anlage nach einem der Ansprüche 12 bis 39, dadurch gekennzeichnet, daß die Mischkörper (62, 57) ebenfalls aus Beton sind.

41. Anlage nach einem der Ansprüche 12 bis 40, dadurch gekennzeichnet, daß im Boden des Gärbehälters (21) ein Sandabschneider (66) eingebaut ist.

**Claims**

1. Process for the production of biogas by fermenting viscous agents in which the fermentation is effected in a fermentation vessel in two stages, a first fermentation stage by acid in which oxygen is consumed by means of acid bacteria, and a subsequent second principal fermentation stage in which the biogas is produced, characterised in that in both fermentation stages the mixing of the fermentation agent is effected by a slow continuous movement in axial direction of the fermentation vessel of approximately plate-shaped mixing bodies (11, 5).

2. Process as claimed in claim 1, characterised in that the fermentation by acid is aerobic.

3. Process as claimed in claim 1, characterised in that the fermentation by acid is anaerobic.

4. Process as claimed in claim 1, characterised in that the exclusive or an additional heating of the fermentation agent is effected by the mixing bodies.

5. Process as claimed in claim 3 or 4, characterised in that the fermentation agent is preheated by means of a heat exchanger before entering the fermentation stage by acid.

6. Process as claimed in claim 2, characterised in that a cooling of the fermentation agent is effected by heat dissipation via the mixing bodies.

7. Process as claimed in claim 2 or 6, characterised in that oxygen is introduced in the fermentation agent by means of the mixing bodies.

8. Process as claimed in any one of claims 1 to 7, characterised in that the supply of the fermentation agent into the fermentation vessel is effected by a pneumatic transport device.

9. Process as claimed in any one of claims 1 to 8, characterised in that by a variation of the speed of the axial movement of the mixing bodies (11, 5) the thickness of the intermixed layer bordering on the mixing bodies is varied.

10. Process as claimed in any one of claims 1 to 9, characterised in that the index of pH of the fermentation agent is measured when leaving the acid fermentation stage.

11. Process as claimed in any one of claims 1 to 10, characterised in that the fermentation agent consists of liquid manure or sewage sludge.

12. Installation for the production of biogas by fermenting viscous agents with a preferably cylindrical vessel in which the fermentation agent is fermented, characterised in that the mixing is effected by approximately plate-shaped mixing bodies (11) which are moved in the vessel (21) in axial direction.

13. Installation as claimed in claim 12, characterised in that the plate-shaped mixing bodies (11) are suspended in a vertical vessel and can be raised and lowered in vertical direction.

14. Installation as claimed in claim 12 or 13, characterised in that the plate-shaped mixing bodies cover the major part of the cross section of the vessel during their axial movement, but there remains a sufficient space between the mixing bodies and the walls of the vessel through which the fermentation agent may flow.

15. Installation as claimed in claim 14, characterised in that the mixing bodies are suspended on ropes and the raising and lowering of the mixing bodies is effected by winding the ropes up and off a roller (8) which is rotatable about its horizontal axis.

16. Installation as claimed in any one of claims 12 to 15, characterised in that the mixing bodies (11, 60, 61) are inclined towards the central axis of the vessel.

17. Installation as claimed in any one of claims 12 to 16, characterised in that the mixing bodies (11, 60) comprise a circumferential upright edge (12) at the margin of the plates with the exception of an inner arcuate region (19) having no edge.

18. Installation as claimed in any one of claims 12 to 17, characterised in that two approximately identical mixing bodies (11, 60, 61) are provided in the vessel which are suspended by means of ropes on the roller (8) in such a manner that the mixing bodies move vertically in opposite directions when the roller is rotated.

19. Installation as claimed in any one of claims 12 to 18, characterised in that the mixing bodies (11, 60, 61) each are suspended on three points in the vessel.

20. Installation as claimed in any one of claims 12 to 19, characterised in that the mixing bodies (11, 60, 61) can be heated or cooled, respectively.

21. Installation as claimed in any one of claims 12 to 20, characterised in that for the heating or the cooling of the mixing bodies (11, 60, 61) water pipes (35) are installed in the latter.

22. Installation as claimed in any one of claims 12 to 21, characterised in that in at least one of the mixing bodies (61) oxygen pipes (67) are installed which communicate with openings in the mixing body so that the fermentation agent can be aerated via the mixing body.

23. Installation as claimed in any one of claims 12 to 22, characterised in that for the oxygen supply of the mixing body (61) flexible trailing conduits are provided.

24. Installation as claimed in any one of claims 12 to 23, characterised in that for the water supply of the mixing bodies (11, 60, 61) flexible trailing conduits are provided.

25. Installation as claimed in any one of claims 12 to 24, characterised in that the mixing bodies (11, 60, 61) comprise a semi-annular or a semicircular circumference.

26. Installation as claimed in any one of claims 12 to 25, characterised in that the mixing bodies (11, 60, 61) are made of concrete.

27. Installation as claimed in any one of claims 12 to 26, characterised in that the vessel (21) comprises an acid fermentation chamber (4) with a gas relief pipe (6) for discharging useless gases, said chamber being connected to a principal fermentation chamber (10) by means of an opening (9) provided near the bottom of the vessel, the discharge conduit (16) for the biogas being provided in the upper region of said principal fermentation chamber.

28. Installation as claimed in any one of claims 12 to 27, characterised in that the acid fermentation chamber (4) is annularly shaped and arranged in the interior close to the axis of the vessel, the principal fermentation chamber (10) concentrically surrounding said acid fermentation chamber.

29. Installation as claimed in any one of claims 12 to 28, characterised in that the vessel is divided by means of a vertical partition wall into two chambers, one of these chambers serving as acid fermentation chamber (50) and the other as principal fermentation chamber (51), the acid fermentation chamber (50) being closed at its top by means of a cover (52).

30. Installation as claimed in any one of claims 12 to 29, charaterised in that the acid fermentation chamber (50) and the principal fermentation chamber (51) have approximately the same volumes.

31. Installation as claimed in any one of claims 12 to 30, characterised in that the supply conduit (2) for the fermentation agent opens in the bottom of the vessel into an interior preheating zone (1), the cylindrical walls of which are formed by a double-walled heat exchanger (3).

32. Installation as claimed in any one of claims 12 to 31, characterised in that the supply pipe (53) for supplying the fermentation agent to the acid fermentation chamber (50) is concentrically surrounded by the discharge pipe (59) for the discharge of the used up fermentation agent from the principal fermentation chamber.

33. Installation as claimed in any one of claims 12 to 32, characterised in that the supply of the fermentation agent is effected by a pneumatic transportation device.

34. Installation as claimed in any one of claims 12 to 33, characterised in that the pneumatic transportation device (22) pumps the fermentation agent by thrusts into the vessel by means of compressed air produced by a compressor.

35. Installation as claimed in any one of claims 12 to 34, characterised in that the conduit (40) for the biogas leads through the vessel (21).

36. Installation as claimed in any one of claims 12 to 35, characterised in that for the intermixing of the fermentation agent in the annular space of the discharge pipe (59) there is provided a further mixing body (62) which is also moved in axial direction.

37. Installation as claimed in any one of claims 12 to 36, characterised in that a further approximately cylindrical mixing body (57) having a conical tip is provided for the supply pipe (53).

38. Installation as claimed in any one of claims 12 to 37, characterised in that the mixing bodies (62, 57) are likewise suspended on ropes and are moved in vertical direction by means of the roller (63).

39. Installation as claimed in any one of claims 12 to 38, characterised in that the mixing body for the mixing of the fermentation agent in the acid fermentation chamber (4) is a floating body (5) to which ropes (27, 28) are fastened, said ropes being guided by lower guide rollers (7) and wound up on the roller (8) so that the floating body can rise to the surface when winding off the ropes.

40. Installation as claimed in any one of claims 12 to 39, characterised in that the mixing bodies (62, 57) are likewise made of concrete.

41. Installation as claimed in any one of claims 12 to 40, characterised in that a sand separator (66) is arranged in the bottom of the fermentation vessel (21).

**Revendications**

1. Procédé de production de biogaz par fermentation d'agents visquex, selon lequel la fermentation est réalisée au cours de deux phases dans une cuve de fermentation; la première phase consiste en une fermentation à l'acide, au cours de laquelle l'oxygène est entièrement consommé à l'aide de bactéries d'acide et le biogaz est ensuite produit au cours de la deuxième phase principale de fermentation, caractérisé en ce que pendant les deux phases de fermentation le mélange des agents de fermentation est effectué par des mouvements lents et continus, en direction de l'axe de la cuve de fermentation, provenant de corps mélangeurs d'une forme d'une plaque (11, 5).

2. Procédé selon la revendication 1, caractérisé en ce que la fermentation à l'acide est aérobie.

3. Procédé selon la revendication 1, caractérisé en ce que la fermentation à l'acide est anaérobie.

4. Procédé selon la revendication 1, caractérisé en ce que le réchauffement exclusif ou complémentaire de l'agent de fermentation est réalisé par l'intermédiaire des corps mélangeurs.

5. Procédé selon la revendication 3 ou 4, caractérisé en ce que l'agent de fermentation est réchauffé par un échangeur thermique, avant d'être introduit dans la phase de fermentation à l'acide.

6. Procédé selon la revendication 2, caractérisé en ce que le refroidissement de l'agent de fermentation est réalisé par dissipation thermique, par l'intermédiaire des corps mélangeurs.

7. Procédé selon la revendication 2 ou 6, caractérisé en ce que l'oxygène est amené dans l'agent de fermentation par l'intermédiaire des corps mélangeurs.

8. Procédé selon une des revendications 1 à 7, caractérisé en ce que l'amenée de l'agent de fermentation dans la cuve de fermentation est effectuée par un système transporteur pneumatique.

9. Procédé selon une des revendications 1 à 8, caractérisé en ce que la modification de la vitesse des mouvements axiaux des corps mélangeurs (11, 5) entraîne une variation de l'épaisseur de la couche malaxée, se trouvant à proximité des corps mélangeurs.

**10.** Procédé selon une des revendications 1 à 9, caractérisé en ce que la valeur potentielle PH de l'agent de fermentation est mesurée en sortant de la phase de fermentation à l'acide.

**11.** Procédé selon une des revendications 1 à 10, caractérisé en ce que l'agent de fermentation consiste en purin ou boues de curage.

**12.** Installation de production de biogaz par fermentad'agents visqueux au moyen d'une cuve, de préférence cylindrique, dans laquelle l'agent de fermentation est amené à fermentation, caractérisée en ce que le mélange est effectué par des corps mélangeurs en forme d'une plaque (11), qui sont mis en mouvement en direction de l'axe de la cuve (21).

**13.** Installation selon la revendication 12, caractérisée en ce que les corps mélangeurs en forme d'une plaque (11) sont suspendus dans une cuve, placée verticalement, et peuvent effectuer des mouvements ascendants et descendants.

**14.** Installation selon la revendication 12 ou 13, caractérisée en ce que les corps mélangeurs en forme d'une plaque couvrent la plus grande partie du volume en coupe de la cuve; entre les corps mélangeurs et les parois de la cuve un espace suffisant reste libre, permettant aux agents de fermentation de circuler librement.

**15.** Installation selon la revendication 14, caractérisée en ce que les corps mélangeurs sont suspendus à des câbles et la montée ou la descente des corps mélangeurs est effectuée par l'enroulement ou le déroulement des câbles sur un tambour cylindrique (8), pivotable sur un axe horizontal.

**16.** Installation selon une des revendications 12 à 15, caractérisée en ce que les corps mélangeurs (11, 60, 61) sont légèrement inclinés vers l'axe central de la cuve.

**17.** Installation selon une des revendications 12 à 16, caractérisée en ce que les corps mélangeurs (11, 60) présentent une arête circulaire placée de chant au bord des plaques, à l'exception de la partie intérieure en forme d'arc de cercle (19) qui ne présente pas d'arête.

**18.** Installation selon une des revendications 12 à 17, caractérisée en ce que deux corps mélangeurs (11, 60, 61), à peu près semblables, sont prévus dans la cuve, ces corps mélangeurs sont suspendus par des câbles à un tambour (8) de telle manière que lorsque le tambour tourne, les corps mélangeurs se déplacent verticalement et respectivement en sens inverse.

**19.** Installation selon une des revendications 12 à 18, caractérisée en ce que chacun des corps mélangeurs (11, 60, 61) est suspendus à trois points différents dans la cuve.

**20.** Installation selon une des revendications 12 à 19, caractérisée en ce que les corps mélangeurs (11, 60, 61) peuvent être chauffés ou refroidis.

**21.** Installation selon une des revendications 12 à 20, caractérisée en ce que pour le chauffage ou le refroidissement des corps mélangeurs (11, 60, 61) des conduites d'eau (35) sont intégrées dans ces derniers.

**22.** Installation selon une des revendications 12 à 21, caractérisée en ce que des conduites d'amenée de l'oxygène (67) sont intégrées dans au moins un des corps mélangeurs (61), et qu'elles sont reliées au corps mélangeur par des ouvertures permettant d'aérer l'agent de fermentation par l'intermédiaire du corps mélangeur.

**23.** Installation selon une des revendications 12 à 22, caractérisée en ce que des tuyaux flexibles d'amenée (68) sont prévus dans le corps mélangeur (61) pour l'approvisionnement en oxygène.

**24.** Installation selon une des revendications 12 à 23, caractérisée en ce que pour l'approvisionnement en eau des corps mélangeurs (11, 60, 61) des tuyaux flexibles d'amenée sont prévus.

**25.** Installation selon une des revendications 12 à 24, caractérisée en ce que les corps mélangeurs (11, 60, 61) présentent un contour toroïdal ou demi-circulaire.

**26.** Installation selon une des revendications 12 à 25, caractérisée en ce que les corps mélangeurs (11, 60, 61) sont en béton.

**27.** Installation selon une des revendications 12 à 26, caractérisée en ce que la cuve (21) présente une chambre de fermentation (4) à l'acide avec une conduite d'évacuation (6) des gaz, permettant d'évacuer les gaz non utilisés, qui est liée à une chambre de fermentation principale (10), par une ouverture (9) se trouvant près du fond de la cuve, la chambre de fermentation principale présentant dans sa partie supérieure une conduite d'amenée (16) du biogaz.

28. Installation selon une des revendications 12 à 27, caractérisée en ce que la chambre de fermentation (4) à l'acide a une forme circulaire et est placée à l'intérieur, près de l'axe de la cuve; la chambre de fermentation principale (10) entoure la chambre de fermentation à l'acide concentriquement.

29. Installation selon une des revendications 12 à 28, caractérisée en ce que la cuve est divisée en deux chambres par une paroi verticale; l'une représente la chambre de fermentation à l'acide (50) et l'autre la chambre de fermentation principale (51); la chambre de fermentation à l'acide (50) est fermée en haut par un couvercle (52).

30. Installation selon une des revendications 12 à 29, caractérisée en ce que la chambre de fermentation à l'acide (50) et la chambre de fermentation principale (51) présentent à peu près le même volume.

31. Installation selon une des revendications 12 à 30, caractérisée en ce que la conduite d'amenée (2) des agents de fermentation est située au fond de la cuve et débouche dans une zone intérieure de réchauffement (1), dont les parois cylindriques sont formées par un échangeur thermique (3) à double paroi.

32. Installation selon une des revendications 12 à 31, caractérisée en ce que la conduite d'amenée (53) conduisant l'agent de fermentation dans la chambre de fermentation à l'acide (50) est entourée concentriquement par le tuyaux d'évacuation (59) permettant d'évacuer l'agent de fermentation utilisé, provenant de la chambre de fermentation principale (10).

33. Installation selon une des revendications 12 à 32, caractérisée en ce que l'amenée de l'agent de fermentation est réalisée à l'aide d'un système transporteur pneumatique.

34. Installation selon une des revendications 12 à 33, caractérisée en ce que le système transporteur pneumatique (22) pompe par poussées l'agent de fermentation dans la cuve, à l'aide d'air comprimé produit par un compresseur.

35. Installation selon une des revendications 12 à 34, caractérisée en ce que la conduite du biogaz (40) est amenée à travers la cuve (21).

36. Installation selon une des revendications 12 à 35, caractérisée en ce qu un corps mélangeur supplémentaire (62) est prévu, qui est également mis en mouvement en direction de l'axe de la cuve pour le mélange de l'agent de fermentation dans l'espace circulaire du tuyau d'évacuation (59).

37. Installation selon une des revendications 12 à 36, caractérisée en ce que la conduite d'amenée (53) est pourvue d'un corps mélangeur supplémentaire (57) en forme de cylindre avec une extrémité conique.

38. Installation selon une des revendications 12 à 37, caractérisée en ce que les corps mélangeurs (62, 57) sont également suspendus à des câbles reliés à un tambour (63) permettant des mouvements verticaux.

39. Installation selon une des revendications 12 à 38, caractérisée en ce que le corps mélangeur prévu pour le malaxage de l'agent de fermentation dans la chambre de fermentation à l'acide (4) est un corps flottant (5), qui est suspendu à des câbles (27, 28), qui sont dirigés par une poulie de guidage (7) et enroulés sur un tambour (8), permettant au corps flottant de rester à la surface lorsque le câble est déroulé.

40. Installation selon une des revendications 12 à 39, caractérisée en ce que les corps mélangeurs (62, 57) sont également en béton.

41. Installation selon une des revendications 12 à 40, caractérisée en ce qu'un dessableur (66) est incorporé dans le fond de la cuve de fermentation (21).

Fig.1

Fig. 2

Fig. 3

Fig. 4

Fig. 5

Fig.6

Fig.7

Fig.8

65

64

53

59

55

61

60

56

21

Fig.9

67

71

68

61